Europäisches Patentamt

European Patent Office

Office européen des brevets

(11) Publication number : **0 477 089 A1**

# (12) EUROPEAN PATENT APPLICATION

(21) Application number : 91402470.8

(22) Date of filing : 17.09.91

(51) Int. Cl.⁵ : **A23L 1/09,** C08B 30/18,
A21D 2/18

(30) Priority : **19.09.90 JP 250703/90**

(43) Date of publication of application :
**25.03.92 Bulletin 92/13**

(84) Designated Contracting States :
**AT BE CH DE DK ES FR GB GR IT LI LU NL SE**

(71) Applicant : **MATSUTANI CHEMICAL
INDUSTRIES CO. LTD.
3-Banchi, 5-chome, Kita Itami
Itami-shi, Hyogo-ken (JP)**

(72) Inventor : **Ohkuma, Kazuhiro
4-7, Yayoigaoka 3-Chome
Sanda-shi, Hyogo-ken (JP)**

Inventor : **Hanno, Yoshio
52-401, Ogino 3-chome
Itami-shi, Hyogo-ken (JP)**
Inventor : **Inada, Kazuyuki
6-89-626, Hobai 2-chome
Takarazuka-shi, Hyogo-ken (JP)**
Inventor : **Matsuda, Isao
717-1, Noma Aza Raifuzuki
Itami-shi, Hyogo-ken (JP)**
Inventor : **Katta, Yasuo
Matsutani Kagaku Dokushinryo, 3-7, Kushiro
4-chome
Kawanishi-shi, Hyogo-ken (JP)**

(74) Representative : **Gillard, Marie-Louise et al
Cabinet Beau de Loménie 55, Rue
d'Amsterdam
F-75008 Paris (FR)**

(54) Process for producing dextrin containing dietary fiber.

(57)    A process for producing a dextrin containing dietary fiber characterized by reacting α-amylase with pyrodextrin having a dietary fiber content of 40 to 60wt.% and prepared by roasting potato starch in the presence of hydrochloric acid serving as a catalyst.

EP 0 477 089 A1

## BACKGROUND OF THE INVENTION

### 1.Field of the invention

The present invention relates to a process for producing a dextrin containing dietary fiber by treating pyrodextrin with an enzyme.

### 2.Description of the Prior Art

As is well known, pyrodextrin is obtained by heating starch at a high temperature. When thus treated, the molecules of starch undergo hydrolysis and re-polymerization to form a complex structure and become soluble in water. Pyrodextrin contains a considerable proportion of indigestible component.

In Japanese dietary practice in recent years, the intake of fibers has decreased greatly, and the deficiency of fibers is mentioned as one of the causes for degenerative diseases. Attention has been directed to the need to take dietary fibers.

In view of the above situation, we have conducted continued research on dietary fibers. In the course of the research, we conceived the totally novel idea of utilizing pyrodextrin as a new dietary fiber although pyrodextrin had never been regarded as such because of its sharp pungent odor and undesirable taste. Consequently, we developed a process for producing from pyrodextrin a dextrin containing a dietary fiber which is satisfactorily usable as such, and have already filed a patent application concerning this process (Unexamined Japanese Patent Publication HEI 2-145169).

On the other hand, although the Proskey method was generally used in the past for quantitatively determining dietary fibers, a high performance liquid chromatographic method (hereinafter referred to as the "new analytical method") has already been developed as an improvement over the Proskey method Ever since the new analytical method was made public, this method has been introduced into wide use especially for quantitatively determining various water-soluble dietary fibers.

We also conducted research on the physiological activities of dextrins containing dietary fibers,and consequently found that these dextrins, which are indigestive, have effects to lower insulin requirements, improve the intestinal function and the serum lipid component and lower blood pressure, and filed a patent application concerning them (Unexamined Japanese Patent Publication HEI 2-145 169). We further found that these physiological effects increase approximately in proportion to the quantity, as determined by the new analytical method, of the dietary fiber present in the dextrin. Accordingly we started research on a process for producing a dextrin which contains a large quantity of dietary fiber as determined by the new analytical method and which can be produced readily in quantities and has a quality desirable for use as a food.

## SUMMARY OF THE INVENTION

It is therefore an object of the present invention to provide such a process.

This object can be accomplished by determining the requirements to be fulfilled by pyrodextrin for use as the material, in other words, selecting a specific material for use, roasting the material under specific conditions, and separating off digestible dextrin by a chromatographic method or organic solvent method when desired.

## DETAILED DESCRIPTION OF THE INVENTION

The pyrodextrin for use in the present invention needs to fulfill the following requirements.
(1) The dextrin is prepared from potato starch in particular among various other starches. (The starch as the material is limited to potato starch.)
(2) The acid catalyst to be used for roasting is hydrochloric acid.
(3) The pyrodextrin contains 40 to 60wt.% of dietary fiber as determined by the new analytical method.

The new analytical method, i.e., the high performance liquid chromatographic method, herein mentioned refers to the following procedure. A 1g quantity of sample is accurately weighed out, 50ml of water is added to the sample, the solution is adjusted to a pH of 5.8, and 0.1ml of $\alpha$-amylase (Termamyl 120L, product of Novo Industry Co., Ltd.) is thereafter added to the solution and reacted therewith at 95°Cfor 30 minutes. The reaction mixture is then cooled and adjusted to a pH of 4.5, 0.1ml of amyloglucosidase (product of Sigma) is added to the mixture and reacted therewith at 60°C for 30 minutes, and the resulting mixture is heated to 90°Cto complete the reaction. The reaction mixture is filtered, the filtrate is concentrated to 5% and then injected to HPLC, and the quantity of glucose produced is determined from the sugar composition. The content of dietary fiber is given by the following equation.

EP 0 477 089 A1

$$\text{Content of dietary fiber}(\%) = 100 - \text{Quantity of glucose produced}(\%)$$

The specified pyrodextrin, when used, affords a food composition having especially important characteristics, i.e., both colorless transparency and a flavor (free from a pungent taste).

Another feature of the present invention is that the desired dextrin obtained by treating the specfic pyrodextrin with $\alpha$-amylase should contain 40 to 60wt.% of dietary fiber. For this reason, white dextrin which has been in wide use for foods and medicaments is not usable since the dietary fiber content of this dextrin is far less than 40wt.% as determined by the new analytical method. Further if the dietary fiber content exceeds 60wt.%, the product dextrin exhibits a pungent taste, while the reaction mixture obtained by the treatment with $\alpha$-amylase will impose an increased load on the subsequent refining step to make mass production impossible.

According to the present invention, the most preferred $\alpha$-amylase for decomposing pyrodextrin is Termamyl (product of Novo Industry Co., Ltd.) or Klaistase T-5 (product of Daiwa Kasei Co., Ltd.) since these enzymes give dextrin of increased dietary fiber content, while other bacterial $\alpha$-amylases are also usable.

The production process of the invention will now be described step by step.

First, pyrodextrin to be used as the starting material is in particular the one prepared from potato starch with use of hydrochloric acid as a catalyst, especially the pyrodextrin prepared from potato starch and containing 40 to 60wt.% of dietary fiber as already stated. To be free from a pungent odor and undesirable taste, the pyrodextrin to be used in the present invention is prepared more preferably by adding an aqueous solution of hydrochloric acid to the starting material starch, pre-drying the mixture to the starting material starch, pre-drying the mixture to a water content of about 5% and roasting the resulting mixture.

Next, the pyrodextrin is dissolved in water to a concentration of 30 to 50wt.%, the solution is adjusted to a pH of 5.5 to 6.5, preferably 5.8, commercial $\alpha$-amylase is added to the solution in an amount of 0.05 to 0.2wt.% based on the pyrodextrin, and the mixture is maintained for 30 minutes to 2 hours at about 85 to about 100°C at which the amylase is active. This causes the enzyme to decompose the dextrin to $\alpha$-limit dextrin. The mixture is then heated to an elevated temperature of 120°C to terminate the enzymatic action of the $\alpha$-amylase.

The mixture obtained by the above reaction is refined as by decolorization with activated carbon and desalting, followed by concentration and spraydrying to obtain a dextrin powder.

According to the present invention, a low-molecular-weight portion can be separated from the mixture resulting from the treatment with $\alpha$-amylase by an ion exchange resin method or organic solvent method. For example, ethanol or like organic solvent can be added to the mixture obtained by decomposition with $\alpha$-amylase to separate off the low-molecular-weight portion.

According to the invention, the above treatment with $\alpha$-amylase can be followed by a further reaction with gluco amylase. This reaction results in the advantage that the fraction other than the dietary fiber can be decomposed to glucose to effectively execute the subsequent separation step. This treatment with gluco amylase is conducted usually under the following conditions. The mixture resulting from the reaction with $\alpha$-amylase is adjusted to a temperature of about 55°C and a pH of 5.5, commercial gluco amylase is added to the mixture in an amount of 0.05 to 0.25wt.% based on the solids and reacted therewith for 20 to 70 hours, and the pH of the resulting mixture is lowered to about 3.5 to deactivate the gluco amylase. The treatment with gluco amylase further facilitates separation of dietary fiber. In this case, the reaction mixture is filtered and refined by conventional procedures, followed by ion exchange resin chromatography or a treatment with an organic solvent to separate off and collect a fraction of dietary fiber, whereby a dextrin can be obtained which contains at least 60% of dietary fiber.

The ion exchange resin chromatography to be employed is such that the dietary fiber fraction can be separated from the low-molecular-weight fraction. For this procedure, a strongly acidic cation exchange resin is used which can be any of those widely used. Preferred examples of such resins are Amberlite IR-116, Amberlite IR-118, Amberlite IR-120B, XT-1022E and XT-471F (all products of Organo Co.), Diaion SK-IB, Diaion SK102, Diaion SK104, Diaion SK106, Diaion SK110, Diaion SK112, Diaion SK116 and Diaion FR01 (all products of Mitsubishi Chemical Industries, Ltd.), XFS-43281.00, XFS-43280.00, XFS-43279.00 and XFS-43278.00 (all products of Dow Chemical Japan).

Before these resins are used for separation, it is usually desirable to use them as the alkali metal type or alkaline earth metal type. Preferably, the mixture is passed through the column at a flow rate adjusted for the particular resin used so as to separate high-molecular-weight dextrin form glucose efficiently. The flow rate is preferably in the range of SV=0.1 to 0.6. Outside this range, the mixture will not be treated or separated efficiently. The mixture is passed through the colum preferably at a temperature of 20 to 70°C. At lower temperatures, the mixture will not be separated efficiently and is likely cause trouble to the resin due to an increased viscosity. If the temperature is higher than 70°C, it is likely that the fraction will become colored brown or other-

3

wise degraded.

The solvent to be used for separation is also such that the low-molecular-weight fraction can be separated off. Examples of useful solvents are ethanol, isopropanol, butanol and like alcohols.

To clarify the features of the present invention, the results or experiments will be described in detail below.

Experimental Example 1

A 50ml quantity of a 1% hydrochloric acid solution was sprayed on 1kg of each of different commercial starches, and the starch was treated in a mixer to obtain a uniform mixture, which was then placed into an aluminum vat, pre-dried in a dryer for 1 hour and subsequently roasted ato 150°C for 2 hours. To the pyrodextrin obtained was added hot water in twice the amount of the pyrodextrin to prepare a solution. The solution was adjusted to a pH of 5.8 with 1N sodium hydroxide solution, 0.1% of Termamyl was added to the solution and reacted therewith at 95°C for 1 hour, and the reaction mixture was heated to 115°C to complete the reaction. Next, the mixture was decolorized, filtered, desalted and concentrated in vacuo to a concentration of 30% by usual methods. Dietary fiber content of pyrodextrin and resulting mixture were quantitatively determined by the new analytical method (method described in EXAMPLES to follow). The transparency of the purified solution was measured by a colorimeter. Table 1 shows the results.

Table 1

| Starch | Dietary fiber content of pyrodextrin(%) | Dietary fiber content of mixture* (%) | Transparency of refined solution |
|---|---|---|---|
| Potato starch | 58.1 | 55.4 | Transparent |
| Tapioca starch | 48.8 | 47.3 | Transparent but red |
| Corn starch | 51.5 | 49.7 | Turbid |

* Mixture resulting from decomposition with $\alpha$-amylase.

Experimental Example 2

A 50ml quantity of 1% hydrochloric acid solution was sprayed on 5kg of commercial potato starch, and the starch was uniformly mixed with the solution by a mixer. The mixture was then placed into an aluminum vat and predried in a dryer for 1 hour. Subsequently, the mixture was roasted at 150°C for 5 hours while collecting a 800g portion of sample from the mixture every hour. To 400g of each of five kinds of pyrodextrins obtained was added hot water in twice the amount of the pyrodextrin to prepare a solution. The solution was adjusted to a pH of 5.8 with 1N sodium hydroxide solution, 0.1% of Termamyl was added to the solution and reacted therewith at 95°C for 1 hour, and the reaction mixture was heated to 115°C to complete the reaction. The reaction mixture was then decolorized and filtered by conventional procedures. The resulting solution was tested by being passed through a mixed bed of ion exchange resins (Amberlite IR-12B and IRA-93). The time when chlorine ion started to leak out into the effluent from the resins during the passage was taken as an end point to determine the amount of effluent passed through the resins. The desalted solution was concentrated in vacuo to a concentration of 30%, the dietary fiber content thereof was quantitatively determined by the new analytical method, and the flavor of the solution was checked by a sensory test.

Table 2 shows the results.

Table 2

| Roasting time (hr) | Amount of effluent passed through ion exchange resins (ml/ml resins) | Dietary fiber content (%) | Flavor (pungent) |
|---|---|---|---|
| 1 | 7.14 | 25.5 | Good |
| 2 | 5.49 | 37.8 | Good |
| 3 | 4.35 | 55.8 | Good |
| 4 | 3.86 | 58.5 | Good |
| 5 | 2.74 | 61.3 | Strong pungent flavor |

Experimental Example 3

A 400g quantity of each pyrodextrin obtained in the above experiment was treated under the same conditions as above except that 0.1% of Klaistase KD was reacted therewith at 85°C, followed by the quantitative determination of the dietary fiber content.
Table 3 shows the results.

Table 3

| Roasting time (hr) | Dietary fiber content (%) |
|---|---|
| 1 | 24.5 |
| 2 | 33.6 |
| 3 | 50.0 |
| 4 | 51.5 |
| 5 | 51.8 |

Table 1 reveals the following. The three kinds of pyrodextrins are over 45% in dietary fiber content. Potato starch, when used, affords a transparent solution, whereas use of tapioca starch produces a red solution. Use of corn starch gives a turbid solution. Accordingly, the latter two starches are unsuitable as materials for beverage use which is one of the important uses of the dextrin of the invention, and it is necessary to use potato starch as the material.

Table 2 indicates that a longer roasting time is desirable since the dietary fiber content increases approximately in proportion to the increase in the roasting time, but the amount of effluent passing through the ion exchange resin decreases when the dietary fiber content is in excess of 60%. Since the treatment with the resin is one of the important refining steps, this result is unsuited to mass production at the factory. Additionally, the dextrin then obtained has a pungent flavor which is unremovable even with the ion exchange resin, and is therefore unusable.

Further a comparison between Tables 2 and 3 with respect to the dietary fiber content shows that the contents listed in Table 2 and resulting from the use of Termamyl are higher, indicating that Termamyl is more preferable to use.

EXAMPLES

The present invention will be described with reference to the following examples, in which the dietary fiber content was determined by the new analytical method as described below.

[Method of Determining Dietary Fiber Content]

A 1g quantity of sample was accurately weighed out, 50ml of water was added to the sample, the solution was adjusted to a pH of 5.8, and 0.1ml of α-amylase (Termamyl 120L, product of Novo industry Co., Ltd.) was thereafter added to the solution and reacted therewith at 95°C for 30 minutes. The reaction mixture was then cooled and adjusted to a pH of 4.5, 0.1ml of amyloglucosidase (product of Sigma) was added to the mixture and reacted therewith at 60°C for 30 minutes, and the resulting mixture was heated to 90°C to complete the reaction. The reaction mixture was filtered, the filtrate was concentrated to 5% and then injected to HPLC, and the quantity of glucose produced was determined from the sugar composition. The dietary fiber content was given by the following equation.

$$\text{Dietary fiber content (\%)} = 100 - \text{Quantity of glucose produced(\%)}$$

## Example 1

Commercial potato starch (2,500kg) was placed into a ribbon mixer, 250 liters of 1% hydrochloric acid solution was sprayed on the starch using compressed air while rotating the mixer, and the mixture was subsequently passed through a pulverizer to obtain a homogenized mixture, which was then aged in the ribbon mixer for 10 hours. The mixture was pre-dried by a flush dryer to a water content of about 3%, thereafter continuously placed into a roaster of the rotary kiln type and roasted at 180°C for 2 hours. The pyrodextrin obtained had a dietary fiber content of 58%. To 2,000kg of the pyrodextrin was added 4,000 liters of water to prepare a solution, which was then adjusted to a pH of 6.0 with 20% sodium hydroxide and hydrolyzed at 95°C for 1 hour with addition of 0.2wt.% of α-amylase (Termamyl 60L, product of Novo Industry Co., Ltd.). A major portion of the reaction mixture was then refined as by decolorization with activated carbon, filtration and desalting with an ion exchange resin, and thereafter spray-dried to prepare about 1,800kg of a dextrin containing 56% of dietary fiber.

## Example 2

The remaining portion (about 100 liters) of the reaction mixture obtained in Example 1 by decomposition with α-amylase was heated to a temperature of 55°C, adjusted to a pH of 5.5 and saccharified for 36 hours with addition of 0.1wt.% of gluco amylase (product of Daiwa Kasei Co., Ltd.), whereupon the pH of the reaction mixture was adjusted to 3.5 to inactivate the gluco amylase. The mixture was then refined in the same manner as in Example 1 and thereafter concentrated to obtain 60kg of 50% solution. The solution (1 liter) was passed at SV=0.25 through a column packed with 50 liters of XFS-43279.00 (product of Dow Chemical Japan) which is a strongly acidic cation exchange resin of the alkali metal type. Subsequently, water was passed through the column to collect a high-molecular-weight dextrin. The effluent contained 92.3% of dietary fiber based on the solids.

## Example 3

To one liter of the 50% concentrate obtained in Example 2 was added 2.5 liters of 95% ethanol with stirring, the mixture was allowed to stand for 1 hour and then centrifuged to separate a precipitate, and the precipitate was dried in vacuo at 70°C to obtain a solid product. The solid product contained 88.6% of dietary fiber.

## Example 4

(Preparation of carbonated beverage tasting like soda pop and containing dietary fiber)

| | |
|---|---|
| Granulated sugar | 120g |
| Citric acid | 1.5g |
| Sodium citrate | 0.1g |
| Vitamin C | 0.18g |
| Soda pop essence | 1.0ml |
| Dextrin obtained in Example 1 | 50g |
| Carbonated water | 550ml |
| Water | 370ml |

The above ingredients were treated in the usual manner to prepare a carbonated beverage containing 2.56% of dietary fiber.

Example 5

(Preparation of orange drink containing dietary fiber)

| | |
|---|---|
| Granulated sugar | 250g |
| Citric acid | 2.3g |
| Malic acid | 1.1g |
| Fruit base | 10ml |
| Colorant | 0.2g |
| Orange essence | 1.9ml |
| Dextrin obtained in Example 2 | 50g |
| Water | 1,500ml |

The above ingredients were treated in the usual manner to prepare an orange drink containing 2.54% of dietary fiber.

Example 6

(Preparation of powdery drink containing dietary fiber)

| | |
|---|---|
| Apple juice powder | 125g |
| Malic acid | 15g |
| Citric acid | 10g |

| Powdery apple flavoring | 10g |
| Frost sugar | 850g |
| Dextrin obtained in Example 1 | 200g |

The above ingredients were uniformly mixed together to prepare a powdery apple juice, 30g of which was dissolved in 200ml of water to prepare a drink. This drink had a dietary fiber content of 2.20%.

## Claims

1. A process for producing a dextrin containing dietary fiber characterized by reacting $\alpha$-amylase with pyrodextrin having a dietary fiber content of 40 to 60wt.% and prepared by roasting potato starch in the presence of hydrochloric acid serving as a catalyst.

2. A process as defined in claim 1 wherein gluco amylase is reacted with the mixture resulting from the reaction with $\alpha$-amylase, and the resulting reaction mixture is filtered and purified in the usual manner and treated by ion exchange resin chromatography or with an organic solvent to separate a dietary fiber fraction from the reaction mixture and obtain a dextrin containing at least 60wt.% of dietary fiber.

European Patent Office

## EUROPEAN SEARCH REPORT

Application Number

EP    91 40 2470

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-368451 (MATSUTANI CHEMICAL INDUSTRIES CO. LTD.)<br>* the whole document * | 1-2 | A23L1/09<br>C08B30/18<br>A21D2/18 |
| D | & JP-A-2145169 | | |
| | --- | | |
| P,X | EP-A-443788 (MATSUTANI CHEMICAL INDUSTRIES CO. LTD.)<br>* claim 3 * | 1-2 | |
| | --- | | |
| P,X | EP-A-443789 (MATSUTANI CHEMICAL INDUSTRIES CO. LTD.)<br>* claim 3 * | 1-2 | |
| | ----- | | |

TECHNICAL FIELDS
SEARCHED (Int. Cl.5 )

A23L
C08B
A21D

The present search report has been drawn up for all claims

| Place of search | Date of completion of the search | Examiner |
|---|---|---|
| BERLIN | 30 OCTOBER 1991 | ALVAREZ ALVAREZ C. |

EPO FORM 1503 03.82 (P0401)